(19) Europäisches Patentamt
European Patent Office
Office européen des brevets

(11) EP 0 602 463 B1

(12) **EUROPÄISCHE PATENTSCHRIFT**

(45) Veröffentlichungstag und Bekanntmachung des Hinweises auf die Patenterteilung:
**13.11.1996 Patentblatt 1996/46**

(51) Int. Cl.$^6$: **C07C 45/50**, C07C 47/02

(21) Anmeldenummer: **93119413.8**

(22) Anmeldetag: **02.12.1993**

(54) **Verfahren zur Herstellung von Aldehyden**

Process for the preparation of aldehydes

Procédé pour la préparation d'aldéhydes

(84) Benannte Vertragsstaaten:
**BE DE ES FR GB IT NL SE**

(30) Priorität: **17.12.1992 DE 4242723**

(43) Veröffentlichungstag der Anmeldung:
**22.06.1994 Patentblatt 1994/25**

(73) Patentinhaber: **HOECHST AKTIENGESELLSCHAFT**
**65926 Frankfurt am Main (DE)**

(72) Erfinder:
- **Bahrmann, Helmut, Dr. Dipl.-Chem.**
  **D-46499 Hamminkeln (DE)**
- **Lappe, Peter, Dr. Dipl.-Chem.**
  **D-46539 Dinslaken (DE)**

(56) Entgegenhaltungen:
**EP-A- 0 107 430**
**WO-A-80/01692**
**FR-A- 2 489 308**
**US-I- B 526 942**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 602 463 B1

## Beschreibung

Die Erfindung betrifft ein Verfahren zur Herstellung von Aldehyden durch Hydroformylierung von Olefinen in Gegenwart wasserlöslicher Rhodium-Komplexkatalysatoren.

Nach einer Variante der seit vielen Jahren industriell in großem Maßstab ausgeübten Oxosynthese erfolgt die Umsetzung von Olefinen mit Synthesegas in Gegenwart einer wässrigen Lösung komplexer Rhodiumverbindungen als Katalysator. Die Grundlagen dieses Prozesses sind in der DE-C-2 627 354 beschrieben, seine technische Ausgestaltung ist Gegenstand der EP-B-103 810. Kennzeichnend für dieses Verfahren ist die Verwendung von Rhodiumverbindungen, die sulfonierte Triarylphosphine als Komplexliganden enthalten. Seine Vorteile gegenüber der homogenkatalysierten Reaktion beruhen vor allem auf der Bildung eines sehr hohen Anteils unverzweigter Aldehyde aus n-Olefinen und der schonenden Abtrennung des Katalysators vom Reaktionsprodukt nach Beendigung der Umsetzung. Sie erfolgt einfach durch Trennung von wässriger und organischer Phase, d.h. ohne Destillation und damit ohne zusätzliche thermische Verfahrensschritte, die zu einer Beeinträchtigung der Aldehydausbeute führen. Neben sulfonierten Triarylphosphinen werden als Komplexbestandteile wasserlöslicher Rhodiumkomplexverbindungen auch carboxylierte Triarylphosphine eingesetzt.

Das Zweiphasenverfahren ist ausgezeichnet zur Hydroformylierung niederer Olefine, insbesondere Ethylen und Propylen` geeignet. Setzt man höhere Olefine wie Hexen, Octen oder Decen ein, geht der Umsatz deutlich zurück, so daß die Wirtschaftlichkeit der Synthese in technischem Maßstab nicht mehr gegeben ist. Verursacht wird der Rückgang des Umsatzes durch die Abnahme der Löslichkeit höherer Olefine in Wasser, denn die Reaktion zwischen den Reaktanten läuft in der wässrigen Phase ab.

Einen Weg, diesen Nachteil zu beheben, eröffnet das in der EP-B-157 316 beschriebene Verfahren. Es besteht darin, bei der Hydroformylierung von Olefinen mit mehr als 6 Kohlenstoffatomen im Molekül in Gegenwart trisulfonierte Triarylphosphine enthaltender Rhodiumkomplexverbindungen als Katalysator, der wässrigen Katalysatorlösung ein quartäres Ammoniumsalz als Lösungsvermittler zuzusetzen. Das Ammoniumion des Salzes enthält einen geradkettigen oder verzweigten Alkyl-, ω-Hydroxyalkyl-, Alkoxy- oder einen gegebenenfalls substituierten Arylrest mit jeweils 6 - 25 Kohlenstoffatomen und 3 gleiche oder verschiedene geradkettige oder verzweigte Alkyl- oder ω-Hydroxyalkylreste mit 1 bis 4 Kohlenstoffatomen.

Eine Weiterentwicklung dieses Verfahrens ist Gegenstand der EP-B-163 234. Nach der Lehre dieser Patentschrift werden $C_6$- bis $C_{20}$-Olefine mit Wasserstoff und Kohlenmonoxid in Gegenwart von Rhodium und einem sulfonierten Arylphosphin umgesetzt, dessen Kation ein quartäres Ammoniumion ist. Das Ammoniumion enthält einen Alkyl- oder Aralkylrest mit 7 bis 18 Kohlenstoffatomen und 3 geradkettige oder verzweigte Alkylreste mit 1 bis 4 Kohlenstoffatomen.

Die beschriebenen Verfahren zur Hydroformylierung höherer Olefine haben sich in der Praxis sehr gut bewährt. Allerdings läßt die Wirksamkeit der als Lösungsvermittler verwendeten quartären Ammoniumsalze über längere Zeiträume noch Wünsche offen, denn sie sind gegenüber Hydrolyse und thermischer Belastung nur begrenzt stabil. Dieses Verhalten hat zur Folge, daß die wässrigen Katalysatorlösungen weniger lange eingesetzt werden können als Lösungen die keinen Lösungsvermittler enthalten.

Es bestand daher die Aufgabe, ein Verfahren zu entwickeln das die aufgezeigten Mängel vermeidet.

Erfindungsgemäß wird die vorstehend beschriebene Aufgabe gelöst durch ein Verfahren zur Herstellung von Aldehyden durch Umsetzung von $C_6$- bis $C_{20}$-Olefinen mit Kohlenmonoxid und Wasserstoff in flüssiger Phase, in Gegenwart einer wässrigen Lösung, die wasserlösliche Rhodium-Phosphin-Komplexverbindungen als Katalysatoren und Lösungsvermittler enthält, bei Temperaturen von 20 bis 150°C und Drücken von 0,1 bis 20 MPa. Es ist dadurch gekennzeichnet, daß die Lösungsvermittler Salze quartärer Phosphoniumverbindungen sind.

Überraschenderweise gelingt die Hydroformylierung höherer Olefine in Gegenwart einer wässrigen Lösung wasserlöslicher Rhodium-Phosphin-Komplexverbindungen als Katalysatoren bei Anwesenheit quartärer Phosphoniumsalze als Lösungsvermittler dauerhaft mit hohem Umsatz und hoher Selektivität. Offensichtlich beeinflussen die Phosphoniumverbindungen nicht das Katalysatorsystem, so daß seine Aktivität erhalten bleibt. Überdies erweisen sich die Phosphoniumverbindungen unter den Bedingungen der Oxosynthese mit wasserlöslichen Katalysatoren als sehr stabil.

Quartäre Phosphoniumverbindungen sind marktgängige Substanzen. Man gewinnt sie in technischem Maßstab, z.B. durch Alkylierung tertiärer Phosphine mit Halogenalkanen. Einen Überblick über weitere Wege zu ihrer Herstellung gibt Houben-Weyl, Methoden der organischen Chemie Band E1 (1982), Seite 491ff.

Bevorzugt werden im erfindungsgemäßen Verfahren Phosphoniumverbindungen eingesetzt, die der allgemeinen Formel

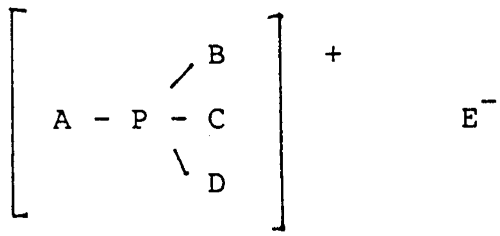

entsprechen. In ihr bedeuten A einen Alkyl- oder Arylrest mit jeweils 6 bis 18 Kohlenstoffatomen oder einen Aralkylrest mit 7 bis 18 Kohlenstoffatomen und B, C und D geradkettige oder verzweigte Alkylreste mit 1 bis 4 Kohlenstoffatomen. E steht für ein Anion und bedeutet Halogenid, Sulfat, Tetrafluoroborat, Acetat, Methosulfat, Benzolsulfonat, Toluolsulfonat, Lactat oder Citrat. Bevorzugte Anionen sind Sulfat, Methosulfat, Sulfonat und Lactat. Die Herstellung von Phosphoniumsalzen mit ausgewählten Anionen erfolgt zweckmäßig über die quartären Phosphoniumhydroxyde. Diese erhält man z.B. aus den Halogeniden, die man in wässriger Lösung durch eine Säule aus stark basischem Anionenaustauscherharz leitet. Ein entsprechendes Verfahren ist z.B. in der DE-C-37 08 041 beschrieben. Zur Durchführung des erfindungsgemäßen Verfahrens geeignete Phosphoniumverbindungen sind z. B. Trimethyltetradecylphosphonium-, Tri-n-butylhexadecylphosphonium-, Tri-n-butylphenylphosphonium- oder Tri-n-butylbenzylphosphoniumsalze.

Die Konzentration der Lösungsvermittler in der wässrigen Katalysatorlösung beträgt 0,5 bis 10 Gew.-% bezogen auf die Katalysatorlösung.

Die als Katalysatoren eingesetzten Rhodiumverbindungen enthalten in komplexer Bindung wasserlösliche Phosphine, d.h. Salze, deren Anion ein Phosphin ist, das mindestens einen sulfonierten oder carboxylierten aromatischen Rest enthält. Der Begriff Phosphin schließt auch solche Verbindungen des dreiwertigen Phosphors ein, in denen das Phosphoratom Bestandteil eines heterocyclischen Ringes ist. Der aromatische Rest kann unmittelbar oder über andere Gruppen an das Phosphoratom des Phosphins gebunden sein. Beispiele für aromatische Reste sind der Phenyl- und der Naphthylrest. Sie können einfach und mehrfach sulfoniert oder carboxyliert und darüberhinaus durch weitere Atomgruppierungen oder Atome wie Alkyl, Hydroxyl, Halogenid substituiert sein. Monophosphinanionen leiten sich bevorzugt von Verbindungen der allgemeinen Formel (1)

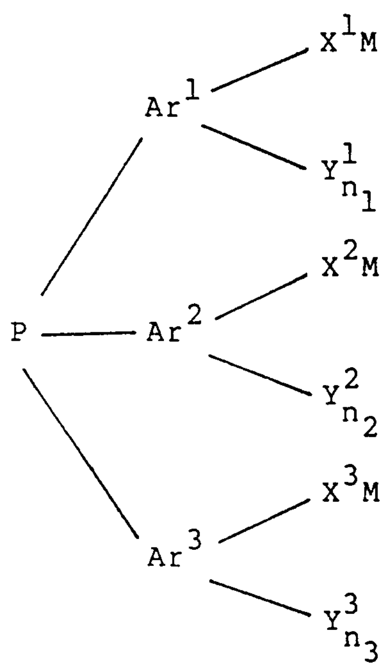

(1)

ab. Hierbei bedeuten $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH-, CN-, $NO_2$- oder $R^1R^2N$-Gruppen, in der $R^1$ und $R^2$ für jeweils eine geradkettige oder verzweigte Alkylgruppe mit 1 bis 4 C-Atomen stehen; $X^1$, $X^2$, $X^3$ ist jeweils ein Carboxylat-($COO^-$-) und/oder ein Sulfonat-($SO_3^-$-)Rest, $n_1$, $n_2$, $n_3$ sind gleiche oder verschiedene ganze Zahlen von 0 bis 5. M ist ein Alkalimetallion, ein Äquivalent eines Erdalkalimetall- oder Zinkions, ein Ammonium-oder quaternäres Ammoniumion der allgemeinen Formel $N(R^3R^4R^5R^6)^+$ in der $R^3$, $R^4$, $R^5$, $R^6$ jeweils für eine geradkettige oder verzweigte Alkylgruppe mit 1 bis 4 C-Atomen steht.

Besonders geeignet sind Verbindungen der vorstehend beschriebenen allgemeinen Formel, in der $Ar^1$, $Ar^2$, $Ar^3$ jeweils einen Phenylrest und $X^1$, $X^2$, $X^3$ jeweils einen Sulfonatrest bedeuten.

Das Anion kann außer von Monophosphinen auch von Polyphosphinen, insbesondere Diphosphinen, die mindestens einen sulfonierten oder carboxylierten aromatischen Rest enthalten, gebildet werden. Diphosphinanionen leiten sich bevorzugt von Diarylverbindungen der allgemeinen Formel (2)

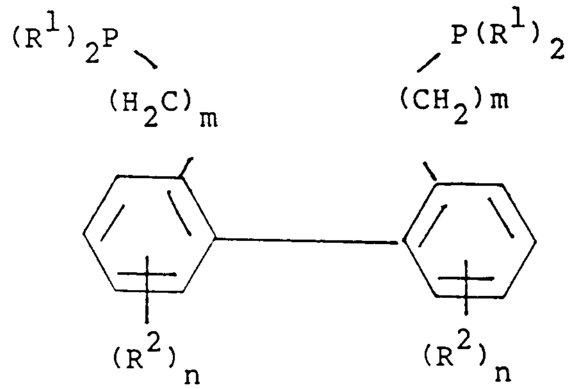

(2)

ab, die durch mindestens einen Sulfonat-($SO_3^-$)-Rest oder Carboxylat-($COO^-$)Rest substituiert sind. In der allgemeinen Formel stehen $R^1$ für gleiche oder verschiedene Alkyl-, Cycloalkyl-, Phenyl-, Tolyl- oder Naphthylreste, $R^2$ ist gleich oder verschieden und bedeutet Wasserstoff, Alkyl- oder Alkoxyreste mit 1 bis 14 Kohlenstoffatomen, ferner Cycloalkyl-, Aryl- oder Aroxyreste mit 6 bis 14 Kohlenstoffatomen oder einen annelierten Benzolring, m ist gleich oder verschieden und steht für ganze Zahlen von 0 bis 5 und n ist ebenfalls gleich oder verschieden und steht für ganze Zahlen von 0 bis 4. Bewährte Vertreter dieser Verbindungsklasse sind die durch Sulfonierung von 2,2'-Bis(diphenylphosphinomethyl)-1,1'-biphenyl oder 2,2'-Bis(diphenylphosphinomethyl)-1,1'-binaphthyl erhaltenen Produkte. Als Beispiel des Anions einer heterocyclischen Phosphorverbindung ist das 3,4-Dimethyl-2,5,6-tris(p-sulfonatophenyl)-1-phosphanorbornadien zu nennen.

Üblicherweise verwendet man als Katalysatorbestandteil die Alkalisalze, insbesondere die Natriumsalze der sulfonierten oder carboxylierten Phosphine. Nach einer bevorzugten Ausführungsform der Erfindung setzt man die Phosphine als Phosphoniumsalze ein. Das Kation ist in diesem Fall ein Phosphoniumion und entspricht dem Kation der Lösungsvermittler. Durch diese Maßnahme verbindet man die katalytischen Eigenschaften der Phosphine mit der lösungsvermittelnden Wirkung der Phosphoniumsalze. Bei dieser Variante des erfindungsgemäßen Verfahrens ist es nicht erforderlich, das gesamte Phosphin als Phosphoniumsalz einzusetzen, vielmehr reicht es aus, lediglich einen Teil des Komplexliganden als Phosphoniumsalz zu verwenden, während der Rest wie üblich als Alkalisalz eingesetzt werden kann.

Die Umsetzung der Olefine mit Wasserstoff und Kohlenmonoxid erfolgt bei Temperaturen von 20 bis 150°C, insbesondere 50 bis 120°C und Drücken von 0,1 bis 20 MPa.

Der Katalysator kann dem Reaktionssystem präformiert zugesetzt werden. Mit gleich gutem Erfolg läßt er sich aber auch aus den Komponenten Rhodium oder Rhodiumverbindung und der wässrigen Lösung des sulfonierten oder carboxylierten Phosphins unter Reaktionsbedingungen im Reaktionsgemisch, also in Gegenwart des Olefins, herstellen. Außer metallischem Rhodium in feinverteilter Form können als Rhodiumquelle wasserlösliche Rhodiumsalze wie Rhodiumchlorid, Rhodiumsulfat, Rhodiumacetat oder in organischen Medien lösliche Verbindungen wie Rhodium-2-ethylhexanoat oder unlösliche Verbindungen wie Rhodiumoxide eingesetzt werden.

Die Rhodiumkonzentration in der wässrigen Katalysatorlösung beträgt 10 bis 2000 Gew.-ppm, bezogen auf die Lösung. Das sulfonierte bzw. carboxylierte Phosphin wird in einer solchen Menge eingesetzt, daß auf 1 mol Rhodium 1 bis 100 mol, vorzugsweise 2 bis 30 mol, Phosphinverbindungen kommen.

Der pH-Wert der wässrigen Katalysatorlösung soll nicht unter 2 liegen. Allgemein stellt man einen pH-Wert von 2 bis 13, vorzugsweise 4 bis 10, ein.

Die Zusammensetzung des Synthesegases, d.h. das Verhältnis von Kohlenmonoxid zu Wasserstoff, kann in weiten Grenzen variiert werden. Im allgemeinen setzt man ein Synthesegas ein, in dem das Volumenverhältnis von Kohlenmonoxid zu Wasserstoff 1 : 1 beträgt oder von diesem Wert nur wenig abweicht.

Die Umsetzung kann sowohl absatzweise als auch kontinuierlich durchgeführt werden.

Das erfindungsgemäße Verfahren wird mit Erfolg bei der Hydroformylierung von geradkettigen oder verzweigten Olefinen, unabhängig von ihrer Molekulargröße, angewandt.

Mit besonderem Erfolg eignet es sich für die Umsetzung von Olefinen, mit sechs und mehr Kohlenstoffatomen. Die Doppelbindung in diesen Olefinen kann end- oder innenständig sein.

Durch folgende Beispiele wird die Erfindung erläutert, nicht aber beschränkt. Zur Charakterisierung der Leistungsfähigkeit der Katalysatorsysteme wird neben dem Verhältnis von n-Aldehyd zu i-Aldehyd der Begriff der "Aktivität" (A-Wert) definiert als

$$\frac{\text{mol Aldehyde}}{\text{mol Rh} \cdot \text{min}}$$

und "Produktivität" (P-Wert) definiert als

$$\frac{\text{g-Aldehyde}}{\text{cm}^3\ \text{Katalysatorlösung} \cdot \text{h}}$$

verwendet. Die Alkohol- und Kohlenwasserstoffbildung ist minimal.

Beispiel 1:

a) Katalysatorpräformierung

In einem 1-1-Autoklav mit Tauchstutzen werden 569 ml einer wäßrigen Lösung, bestehend aus 212 g Trinatrium-tri(m-sulfophenyl)phosphin (0,12 mol) und 382 g Wasser sowie 200 ppm Rh als Rhodiumacetat vorgelegt. Synthesegas ($CO/H_2$-Vol.-Verhältnis 1:1) wird bis zu einem Druck von 25 bar (2,5 MPa) aufgepreßt. Darauf wird die Reaktionslösung drei Stunden unter Rühren bei 110°C mit Synthesegas behandelt, wobei sich der aktive Katalysator bildet. Nach dem Abkühlen auf etwa 30°C wird die Rührung abgestellt und nach einer Absetzzeit von 15 Minuten die überschüssige Lösung (etwa 80 g) über den Tauchstutzen entnommen und analysiert. Die restliche Lösung verbleibt im Reaktor.

b) Hydroformylierung

In die nach a) hergestellte Lösung werden unter Rühren 250 g Tetradecen-1 gepumpt. Bei konstantem Druck von 2,5 MPa wird das Gemisch auf 110°C erhitzt und 6 Stunden bei dieser Temperatur belassen. Danach kühlt man auf 70°C ab und läßt absitzen. Die überstehende organische Phase wird über den Tauchstutzen entnommen. Sie wird gewogen und gaschromatografisch untersucht. Der Teilschritt b) wird insgesamt zweimal wiederholt. Die Ergebnisse dieser Versuche (1.1, 1.2, 1.3) sind in der Tabelle aufgeführt. Der Umsatz liegt bei lediglich 0,1 %. Die Werte für Aktivität und Produktivität beziehen sich auf die im Autoklaven vorhandenen Mengen wäßriger und organischer Phase. Die Dichte der wäßrigen Phase beträgt 1,055 $g/cm^3$.

Beispiel 2

a) Katalysatorpräformierung

In einem 1-1 Autoklaven mit Tauchstutzen werden 569 ml einer wäßrigen Lösung bestehend aus 212 g Trinatrium-tri(m-sulfophenyl)phosphin (0,12 mol), 15,8 g Tetradecyl-triethylphosphoniumbromid, 30 g Pufferlösung (pH 6,0) und 336 g Wasser sowie 200 ppm Rh als Rhodiumacetat vorgelegt. Synthesegas ($CO/H_2$= 1:1) wird bis zu einem Druck von 2,5 MPa aufgepreßt. Danach wird die Reaktionslösung drei Stunden unter Rühren bei 110°C mit Synthesegas behandelt, wobei sich der aktive Katalysator bildet. Nach dem Abkühlen auf etwa 30°C wird die Rührung abgestellt und nach einer Absetzzeit von 15 Minuten die überschüssige Lösung (etwa 80 g) über den Tauchstutzen entnommen und analysiert. Die restliche Lösung verbleibt im Autoklaven.

b) Hydroformylierung

In die nach a) hergestellte Lösung werden unter Rühren 250 g Tetradecen-1 gepumpt. Bei konstantem Druck von 2,5 MPa wird das Gemisch auf 110 °C erhitzt und 6 Stunden bei dieser Temperatur belassen. Danach kühlt man auf 70°C ab und läßt absitzen. Die überstehende organische Phase wird über den Tauchstutzen entnommen. Sie wird gewogen und gaschromatografisch untersucht. Der Teilschritt b) wird insgesamt viermal wiederholt. Die Ergebnisse dieser Versuche (2.1, 2.2, 2.3, 2.4, 2.5) sind in der Tabelle aufgeführt. Der Umsatz steigt stark an und stabilisiert sich auf ermäßigtem Niveau. Die Werte für Aktivität und Produktivität beziehen sich auf die im Autoklaven vorhandenen Mengen wäßriger und organischer Phase. Die Dichte der wäßrigen Phase beträgt 1,057 $g/cm^3$.

Beispiel 3

a) Katalysatorpräformierung

In einem 1-1 Autoklaven mit Tauchstutzen werden 569 ml einer wäßrigen Lösung, bestehend aus 212 g Trinatrium-tri(m-sulfophenyl)phosphin(0,12 mol), 20,4 g Tributyl-hexadecylphosphoniumbromid, 30 g Pufferlösung (pH 6,0) und 332 g Wasser sowie 200 ppm Rh als Rhodiumacetat vorgelegt. Synthesegas ($CO/H_2$-Vol.-Verhältnis 1:1) wird bis zu einem Druck von 2,5 MPa aufgepreßt. Danach wird die Reaktionslösung drei Stunden unter Rühren bei 110°C mit Synthesegas behandelt, wobei sich der aktive Katalysator bildet. Nach dem Abkühlen auf etwa 30°C wird die Rührung abgestellt und nach einer Absetzzeit von 15 Minuten die überschüssige Lösung (etwa 80 g) über den Tauchstutzen entnommen und analysiert. Die restliche Lösung bleibt im Autoklaven.

b) Hydroformylierung

In die nach a) hergestellte Lösung werden unter Rühren 250 g Tetradecen-1 gepumpt. Bei konstantem Druck von 2,5 MPa wird das Gemisch auf 110°C erhitzt und 6 Stunden bei dieser Temperatur belassen. Danach kühlt man auf 70 °C ab und läßt absitzen. Die überstehende organische Phase wird über den Tauchstutzen entnommen. Sie wird gewogen und gaschromatografisch untersucht. Der Teilschritt b) wird insgesamt dreimal wiederholt. Die Ergebnisse dieser Versuche (3.1, 3.2, 3.3, 3.4) sind in der Tabelle aufgeführt. Der Umsatz stabilisiert sich gegenüber dem Nullversuch auf erhöhtem Niveau. Die Werte für Aktivität und Produktivität beziehen sich auf die im Autoklaven verbliebenen Mengen wäßriger und organischer Phase. Die Dichte der wäßrigen Phase beträgt 1,059 $g/cm^3$.

Tabelle

| | Beispiel 1 | | | Beispiel 2 | | | | | Beispiel 3 | | | |
|---|---|---|---|---|---|---|---|---|---|---|---|---|
| | 1.1 | 1.2 | 1.3 | 2.1 | 2.2 | 2.3 | 2.4 | 2.5 | 3.1 | 3.2 | 3.3 | 3.4 |
| Umsatz (nach GC*) | 0.10 | 0.10 | 0.11 | 74.2 | 48.6 | 42.1 | 37.0 | 28.4 | 34.1 | 8.8 | 5.3 | 5.8 |
| A-Wert | 0.00 | 0.00 | 0.00 | 3.10 | 1.66 | 1.32 | 1.13 | 1.01 | 1.17 | 0.21 | 0.11 | 0.11 |
| P-Wert | 0.00 | 0.00 | 0.00 | 0.075 | 0.04 | 0.032 | 0.027 | 0.024 | 0.028 | 0.005 | 0.003 | 0.003 |

* Gaschromatographie

**Patentansprüche**

1. Verfahren zur Herstellung von Aldehyden durch Umsetzung von $C_6$- bis $C_{20}$-Olefinen mit Kohlenmonoxid und Wasserstoff in flüssiger Phase, in Gegenwart einer wäßrigen Lösung, die wasserlösliche Rhodium-Phosphin-Komplexverbindungen als Katalysatoren und Lösungvermittler enthält, dadurch gekennzeichnet, daß die Lösungsvermittler Salze quarternärer Phosphoniumverbindungen sind.

2. Verfahren nach Anspruch 1, dadurch gekennzeichnet, daß die Phosphoniumsalze der allgemeinen Formel

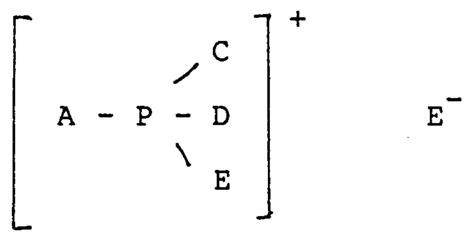

entsprechen und A einen Alkyl- oder Arylrest mit jeweils 6 bis 18 Kohlenstoffatomen und B, C und D geradkettige oder verzweigte Alkylreste mit 1 bis 4 Kohlenstoffatomen bedeuten und E für ein Anion steht.

3. Verfahren nach Anspruch 2, dadurch gekennzeichnet, daß das Anion des Phosphoniumsalzes ein Halogenid, Sulfat, Tetrafluoroborat, Acetat, Methosulfat, Benzolsulfonat, Toluolsulfonat, Lactat oder Citrat ist.

4. Verfahren nach Anspruch 2, dadurch gekennzeichnet, daß das Anion des Phosphoniumsalzes das als Bestandteil der Komplexverbindung verwendete sulfonierte oder carboxylierte Phosphin ist.

5. Verfahren nach einem oder mehreren der Ansprüche 1 bis 4, dadurch gekennzeichnet, daß die als Katalysatoren eingesetzten Rhodiumverbindungen in komplexer Bindung trisulfonierte bzw. tricarboxylierte Triarylphosphine der allgemeinen Formel

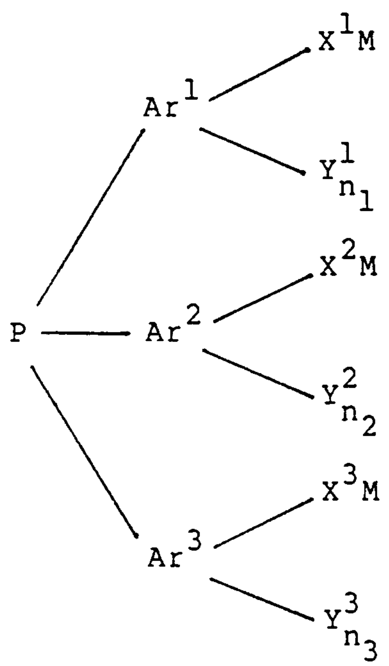

(1)

enthalten, wobei $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH-, CN-, $NO_2$- oder $R^1R^2N$-Gruppen, in der $R^1$ und $R^2$ für jeweils eine geradkettige oder verzweigte Alkylgruppe mit 1 bis 4 C-Atomen stehen bedeuten, $X^1$, $X^2$, $X^3$ jeweils ein Carboxylat-($COO^-$-) und/oder ein Sulfonat-($SO_3^-$-)Rest ist, $n_1$, $n_2$, $n_3$ gleiche oder verschiedene ganze Zahlen von 0 bis 5 sind und M ein Alkali-, Erdalkalimetall-, Zink-, Ammonium- oder quaternäres Ammoniumion der allgemeinen Formel $N(R^3R^4R^5R^6)^+$ in der $R^3$, $R^4$, $R^5$, $R^6$ jeweils für eine geradkettige oder verzweigte Alkylgruppe mit 1 bis 4 C-Atomen steht, ist.

6. Verfahren nach Anspruch 5, dadurch gekennzeichnet, daß $Ar^1$, $Ar^2$, $Ar^3$ jeweils einen Phenylrest und $X^1$, $X^2$, $X^3$ jeweils einen Sulfonatrest bedeuten.

7. Verfahren nach einem oder mehreren der Ansprüche 1 bis 5, dadurch gekennzeichnet, daß die als Katalysatoren eingesetzten Rhodiumverbindungen in komplexer Bindung Diarylverbindungen der allgemeinen Formel

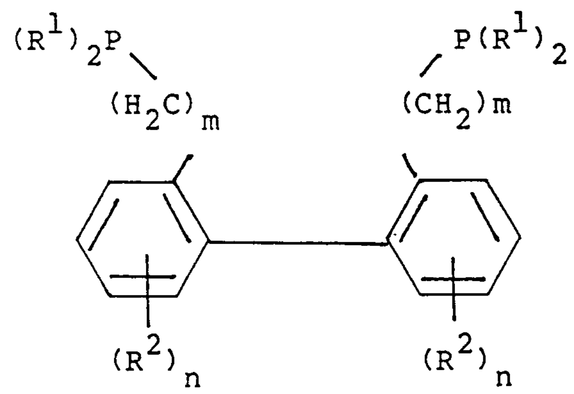

die durch mindestens einen Sulfonat-($SO_3^-$)-Rest oder Carboxylat-($COO^-$)Rest substituiert sind enthalten, wobei $R^1$ für gleiche oder verschiedene Alkyl-, Cycloalkyl-, Phenyl-, Tolyl- oder Naphthylreste steht, $R^2$ gleich oder verschieden ist und Wasserstoff, Alkyl- oder Alkoxyreste mit 1 bis 14 Kohlenstoffatomen, ferner Cycloalkyl-, Aryl- oder Aroxyreste mit 6 bis 14 Kohlenstoffatomen oder einen annelierten Benzolring bedeutet, m gleich oder verschieden ist und für ganze Zahlen von 0 bis 5 und n ebenfalls gleich oder verschieden ist und für ganze Zahlen von 0 bis 4 steht.

8. Verfahren nach einem oder mehreren der Ansprüche 1 bis 7, dadurch gekennzeichnet, daß die Konzentration des Lösungsvermittlers in der wäßrigen Katalysatorlösung 0,5 bis 10 Gew.-%, bezogen auf die Katalysatorlösung, beträgt.

## Claims

1. A process for the preparation of aldehydes by reaction of $C_6$- to $C_{20}$-olefins with carbon monoxide and hydrogen in liquid phase in the presence of an aqueous solution containing water-soluble rhodium-phosphine complex compounds as catalysts and containing solubilizers, wherein the solubilizers are salts of quaternary phosphonium compounds.

2. The process as claimed in claim 1, wherein the phosphonium salts are of the formula

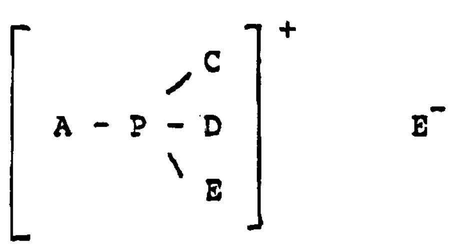

in which A is an alkyl or aryl radical having in each case from 6 to 18 carbon atoms and B, C and D are straight-chain or branched alkyl radicals having from 1 to 4 carbon atoms and E represents an anion.

3. The process as claimed in claim 2, wherein the anion of the phosphonium salt is a halide, sulfate, tetrafluoroborate, acetate, methosulfate, benzene-sulfonate, toluenesulfonate, lactate or citrate.

4. The process as claimed in claim 2, wherein the anion of the phosphonium salt is the sulfonated or carboxylated phosphine used as a component of the complex compound.

5. The process as claimed in one or more of claims 1 to 4, wherein the rhodium compounds employed as catalysts contain, bound as a complex, trisulfonated and/or tricarboxylated triarylphosphines of the formula

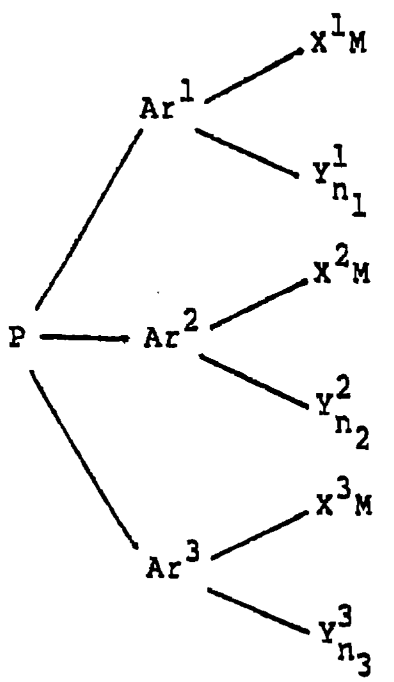

(1)

in which $Ar^1$, $Ar^2$ and $Ar^3$ are each a phenyl or naphthyl group, $Y^1$, $Y^2$ and $Y^3$ are each a straight-chain or branched alkyl group having from 1 to 4 carbon atoms, an alkoxy group, a halogen atom, the groups OH, CN, $NO_2$ or $R^1R^2N$, in which $R^1$ and $R^2$ are each a straight-chain or branched alkyl group having from 1 to 4 carbon atoms, $X^1$, $X^2$ and $X^3$ are in each case a carboxylate ($COO^-$) and/or a sulfonate ($SO_3^-$) radical, and $n_1$, $n_2$ and $n_3$ are identical or different integers from 0 to 5, and M is an alkali metal, alkaline earth metal, zinc, ammonium or quaternary ammonium ion of the formula $N(R^3R^4R^5R^6)^+$ in which $R^3$, $R^4$, $R^5$ and $R^6$ are each a straight-chain or branched alkyl group having from 1 to 4 carbon atoms.

6. The process as claimed in claim 5, wherein $Ar^1$, $Ar^2$ and $Ar^3$ are each a phenyl radical and $X^1$, $X^2$ and $X^3$ are each a sulfonate radical.

7. The process as claimed in one or more of claims 1 to 5, wherein the rhodium compounds employed as catalysts contain, bound as a complex, diaryl compounds of the formula

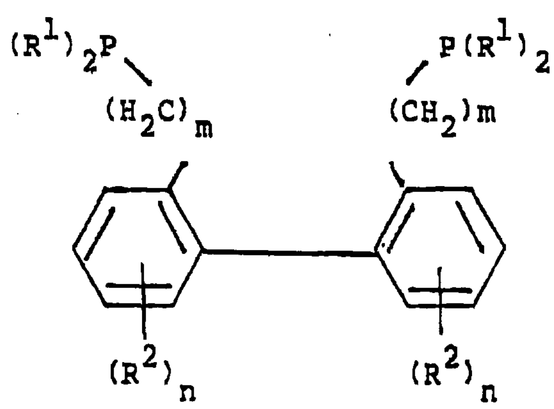

which are substituted by at least one sulfonate ($SO_3^-$) radical or carboxylate ($COO^-$) radical, in which, at each occurrence, $R^1$ is an identical or different alkyl, cycloalkyl, phenyl, tolyl or naphthyl radical, $R^2$ is identical or different and is hydrogen, an alkyl or alkoxy radical having from 1 to 14 carbon atoms, or is a cycloalkyl, aryl or aroxy radical having from 6 to 14 carbon atoms, or is a fused-on benzene ring, m is identical or different and is an integer from 0 to 5, and n is likewise identical or different and is an integer from 0 to 4.

**8.** A process as claimed in one or more of claims 1 to 7, wherein the concentration of the solubilizer in the aqueous catalyst solution is from 0.5 to 10% by weight, based on the catalyst solution.

## Revendications

**1.** Procédé de préparation d'aldéhydes par réaction d'oléfines en $C_6$-$C_{20}$ avec l'oxyde de carbone et l'hydrogène en phase liquide en présence d'une solution aqueuse contenant des complexes rhodium-phosphines solubles dans l'eau qui servent de catalyseurs et d'agents solubilisants, caractérisé en ce que les agents solubilisants sont des sels de dérivés de phosphonium quaternaire.

**2.** Procédé selon la revendication 1, caractérisé en ce que les sels de phosphonium répondent à la formule générale

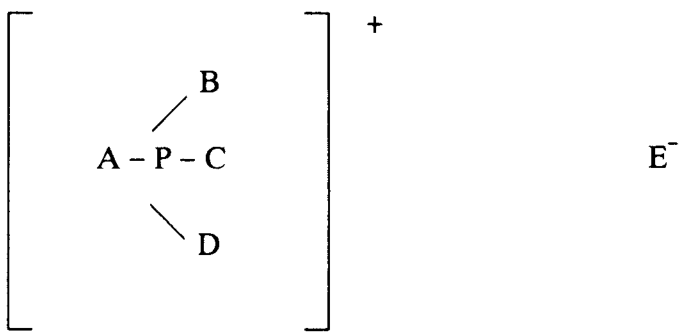

dans laquelle A représente un groupe alkyle ou aryle en $C_6$-$C_{18}$ et B, C et D représentent des groupes alkyles à chaîne droite ou ramifiée en $C_1$-$C_4$, et E est un anion.

**3.** Procédé selon la revendication 2, caractérisé en ce que l'anion du sel de phosphonium est un anion halogénure, sulfate, tétrafluoroborate, acétate, méthylsulfate, benzènesulfonate, toluènesulfonate, lactate ou citrate.

**4.** Procédé selon la revendication 2, caractérisé en ce que l'anion du sel de phosphonium consiste en la phosphine sulfonée ou carboxylée utilisée en tant que constituant du complexe.

**5.** Procédé selon l'une ou plusieurs des revendications 1 à 4, caractérisé en ce que les dérivés du rhodium utilisés en tant que catalyseurs contiennent en liaison complexe des triarylphosphines trisulfonées ou tricarboxylées de formule générale

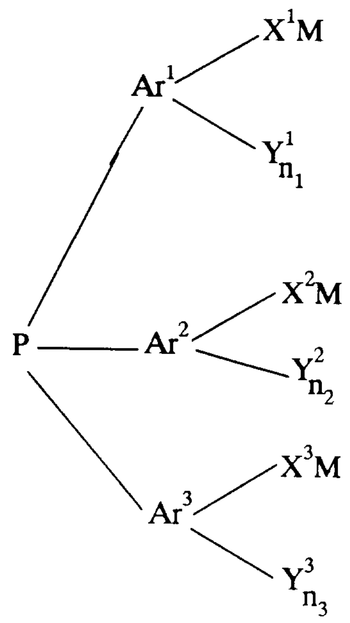

(1)

dans laquelle $Ar^1$, $Ar^2$, $Ar^3$ représentent chacun un groupe phényle ou naphtyle, $Y^1$, $Y^2$, $Y^3$ représentent chacun un groupe alkyle à chaîne droite ou ramifiée en $C_1$-$C_4$, un groupe alcoxy, un atome d'halogène, un groupe OH, CN, $NO_2$ ou $R^1R^2N$ dans lequel $R^1$ et $R^2$ représentent chacun un groupe alkyle à chaîne droite ou ramifiée en $C_1$-$C_4$ ; $X^1$, $X^2$, $X^3$ représentent chacun un groupe carboxylate ($COO^-$) et/ou sulfonate ($SO_3^-$), $n_1$, $n_2$, $n_3$ sont des nombres entiers identiques ou différents allant de 0 à 5, M est un ion de métal alcalin, alcalino-terreux, de zinc, d'ammonium ou d'ammonium quaternaire de formule générale $N(R^3R^4,R^5R^6)^+$ dans laquelle $R^3$, $R^4$, $R^5$, $R^6$ représentent chacun un groupe alkyle à chaîne droite ou ramifiée en $C_1$-$C_4$.

6. Procédé selon la revendication 5, caractérisé en ce que $Ar^1$, $Ar^2$, $Ar^3$ représentent chacun un groupe phényle et $X^1$, $X^2$, $X^3$ représentent chacun un groupe sulfonate.

7. Procédé selon l'une ou plusieurs des revendications 1 à 5, caractérisé en ce que les dérivés du rhodium utilisés en tant que catalyseurs contiennent en liaison complexe des dérivés diaryliques de formule générale

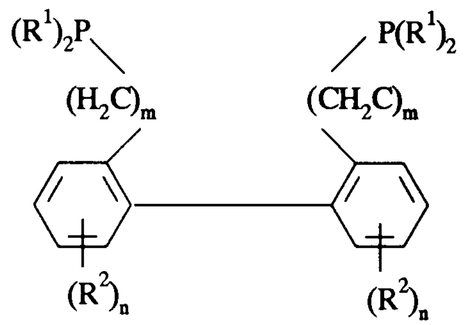

(2)

substitués par au moins un groupe sulfonate ($SO_3^-$) ou carboxylate ($COO^-$), et les symboles $R^1$, ayant des significations identiques ou différentes, représentent chacun un groupe alkyle, cycloalkyle, phényle, tolyle ou naphtyle, les symboles $R^2$, ayant des significations identiques ou différentes, représentent chacun l'hydrogène, un groupe alkyle ou alcoxy en $C_1$-$C_{14}$, ou un groupe cycloalkyle, aryle ou aroxy en $C_6$-$C_{14}$, ou bien un cycle benzénique condensé, les indices m, identiques ou différents, sont chacun des nombres entiers allant de 0 à 5 et les indices n, également identiques ou différents, sont chacun des nombres entiers allant de 0 à 4.

8. Procédé selon une ou plusieurs des revendications 1 à 7, caractérisé en ce que la concentration de l'agent solubilisant dans la solution aqueuse de catalyseur est de 0,5 à 10 % du poids de la solution de catalyseur.